Europäisches Patentamt

European Patent Office

Office européen des brevets

(19)

(11) Numéro de publication : **0 314 744 B1**

(12) # FASCICULE DE BREVET EUROPEEN

(45) Date de publication du fascicule du brevet :
**19.08.92 Bulletin 92/34**

(51) Int. Cl.⁵ : **G01N 33/68,** G01N 33/577,
G01N 33/543

(21) Numéro de dépôt : **88904517.5**

(22) Date de dépôt : **18.05.88**

(86) Numéro de dépôt international :
**PCT/EP88/00430**

(87) Numéro de publication internationale :
**WO 88/09508 01.12.88 Gazette 88/26**

(54) **DOSAGE DE MONOKINES.**

(30) Priorité : **19.05.87 FR 8706988**

(43) Date de publication de la demande :
**10.05.89 Bulletin 89/19**

(45) Mention de la délivrance du brevet :
**19.08.92 Bulletin 92/34**

(84) Etats contractants désignés :
**AT BE CH DE FR GB IT LI LU NL SE**

(56) Documents cités :
**EP-A- 0 220 063**
**WO-A-83/04312**
**FR-A- 2 588 476**
**US-A- 4 302 437**
**JOURNAL OF CLINICAL LABORATORY ANA-LYSIS, vol. 1, 1987, Alain R. Liss Inc.; M.LYTE, pp. 83-88**
**THE JOURNAL OF IMMUNOLOGY, vol. 138, no. 12, 15 June 1987, The American Association of Immunologists (US); J.S.KENNEY et al,pp. 436-4242**
**BIOTECHNIQUES, vol. 5, no. 7, October 1987; E.W.GAFFNEY et al., pp. 652-657**
**THE JOURNAL OF IMMUNOLOGY, vol. 137, no. 8, 15 October 1986, The American Association of Immunologists (US); P.M.PETERS etal, pp. 2592-2598**
**LYMPHOKINE RESEARCH, vol. 6, no. 3, 3rd quarter of 1987, Mary Ann Liebert Inc., Publishers; P.J.LISI et al, pp. 229-233**

(56) Documents cités :
**THE JOURNAL OF IMMUNOLOGY, vol. 137, no. 8, 15 October 1986, The American Association of Immunologists (US); R.S.KORNBLUTH etal, pp. 2585-2591**
**JOURNAL OF IMMUNOLOGICAL METHODS, vol. 83, no. 1, 1985, Elsevier Science Publishers V.V., Amsterdam (NL); A.J.H.GEARING etal, pp. 1-27**

(73) Titulaire : **MEDGENIX DIAGNOSTIC**
**B-6220 Fleurus (BE)**

(72) Inventeur : **DELACROIX, Dominique**
**11, avenue des Ramiers**
**B-1950 Kraainem (BE)**
Inventeur : **DE GROOTE, Donat**
**32, avenue Valentin Tondeur**
**B-1410 Waterloo (BE)**
Inventeur : **FRANCHIMONT, Paul**
**17, rue du Village**
**B-5280 Modave (BE)**
Inventeur : **GYSEN, Philippe**
**10, avenue des Pins**
**B-4121 Neupre (BE)**
Inventeur : **REUTER, Aimée**
**17/022, bd Frère Orban**
**B-4000 Liège (BE)**
Inventeur : **DEHART, Isabelle**
**189, rue Louis Hap**
**B-1040 Bruxelles (BE)**

(74) Mandataire : **Warcoin, Jacques et al**
**Cabinet Régimbeau 26, avenue Kléber**
**F-75116 Paris (FR)**

## Description

La présente invention concerne, d'une manière générale, les dosages de monokines. Plus précisément, la présente invention concerne le dosage de monokines endogènes dans un échantillon sanguin.

Les monokines font partie de l'ensemble des médiateurs cellulaires que sont les cytokines. Parmi les cytokines, on distingue en effet

– les lymphokines, médiateurs solubles sécrétés par les lymphocytes et activant soit les macrophages, soit d'autres lymphocytes, et

– les monokines, médiateurs sécrétés par les monocytes ou les macrophages et agissant soit sur les cellules de la lignée lymphocytaire, soit sur des cellules d'origine mésenchymateuses.

Les monokines concernées par la présente invention consistent notamment dans le "tumour nécrosis factor" (TNF$\alpha$) et l'interleukine 1 (IL-1$\beta$).Ces deux médiateurs cellulaires sont en effet connus pour présenter des similitudes marquées dans leur comportement tel que leur cinétique de libération ou dans leur action biologique ((1) voir références bibliographiques).

Outre leur aspect de facteurs anticancéreux ou antiviral, on sait que le TNF$\alpha$ et l'IL-1$\beta$ sont sécrétés dans différentes pathologies, particulièrement pour le TNF$\alpha$, dans les états inflammatoires et lors de chocs septiques ou pancréatiques. D'une manière plus générale, la sécrétion de TNF$\alpha$ et d'IL-1$\beta$ révèle une activation macrophagique. Par conséquent, la possibilité de suivre cette sécrétion de TNF$\alpha$ ou d'IL-1$\beta$ par un dosage simple, fiable et rapide peut être une source de renseignements très révélatrice concernant l'aspect fonctionnel des macrophages dans de nombreuses situations pathologiques.

Lorsque l'on envisage le dosage immunologique de cytokines dans un échantillon sanguin, on rencontre certaines difficultés. Souvent la sensibilité des dosages est considérée comme insuffisante pour détecter ces médiateurs directement dans le plasma ou dans le sérum sanguin, les quantités présentes à doser étant trop faibles. La difficulté peut aussi tenir au caractère difficilement exploitable des valeurs obtenues, le cas échéant, les valeurs sériques ou plasmatiques variant peu ou de manière non significative de sujet al sujet, ou entre sujets sains et sujets malades. Ceci est particulièrement le cas des lymphokines (IFN$\gamma$ (2) et IL-2 (3 a-b)).

S'agissant du TNF$\alpha$, comme on le verra plus loin (exemple V : évaluation clinique), le dosage immunologique RIA qui est pourtant très sensible ne permet pas de détecter directement dans le plasma des concentrations inférieures à 20 pg/ml, et ceci pour des durées d'incubation de dosage de 24 heures. Si, de surcroît, on réduit cette durée à 4 heures, ce qui est plus conforme aux exigences pratiques, cette concentration limite de détection s'élève jusqu'al environ 100 pg/ml.

Quant aux dosages IRMA, qui requièrent des incubations de seulement 2 heures, ils ne permettent pas toutefois de détecter directement la présence de monokines dans le plasma.

Les concentrations de TNF$\alpha$, obtenues par dosage RIA direct dans le plasma, sont en moyenne pour des sujets sains de l'ordre de 50 pg/ml et ne dépassent pas 200 pg/ml dans la plupart des pathologies. Ces dosages RIA directs dans le plasma sont cependant difficilement exploitables.

On obtient en effet des valeurs qui varient peu d'un sujet à l'autre, même si la différence peut être significative entre sujet sain et sujet atteint d'une pathologie dans certains cas. En outre, on considère en général que les quantités à doser doivent être nettement supérieures (4 à 5 fois au moins) à la valeur limite de détection du dosage pour offrir une finesse satisfaisante de dosage, c'est-al-dire la possibilité de variations importantes de sujet à sujet. Ces deux conditions ne sont donc pas respectées.

Traditionnellement, la solution a consisté à doser les cytokines d'un échantillon sanguin en ayant recours à une étape de culture cellulaire pour stimuler la sécrétion des cytokines ((2) IFN$\gamma$, (3 a-b) IL-2, (4) TNF$\alpha$, (5) IL-1).

L'article de "Journal of Clinical Laboratory Analysis", volume 1 (1987), pages 83-88, décrit un dosage d'interleukines 1 et d'interleukines 2 dans lequel il est recouru à une étape de culture du sang total de 24 heures dans un milieu RPMI à 37°C en présence de $CO_2$.

Les dosages consistent alors à doser la libération de cytokines dans des milieux de culture stimulés par l'adjonction d'un agent mitogène, par exemple à doser les monokines sur le surnageant de monocytes du sang périphérique mis en culture en présence d'agent mitogène. On arrive alors à des valeurs de l'ordre du nanogramme par ml dans le cas du TNF$\alpha$ (voir exemple V ), c'est-à-dire des concentrations nettement supérieures à la limite de détection des dosages et présentant des variations importantes d'un sujet à l'autre, surtout lorsqu'on compare sujets normaux et sujets pathologiques.

Cependant, il est évident que ce recours à une étape de culture cellulaire est une solution peu satisfaisante, notamment dans la perspective de l'application du dosage en routine clinique. L'étape de culture entraîne en effet certaines variations qui affectent la fiabilité du dosage, notamment sa reproductibilité de patient à patient d'une part, mais aussi de prélèvement à prélèvement pour un même patient, d'autre part, sans compter en outre la nécessité d'une intrastructure de culture, de conditions de stérilité, ou encore une durée de dosage assez longue.

Un but de la présente invention était donc de proposer une méthode de dosage immunologique de monokines dans un échantillon sanguin qui soit fiable et exploitable quant aux valeurs obtenues, mais simple et facilement utilisable en routine clinique.

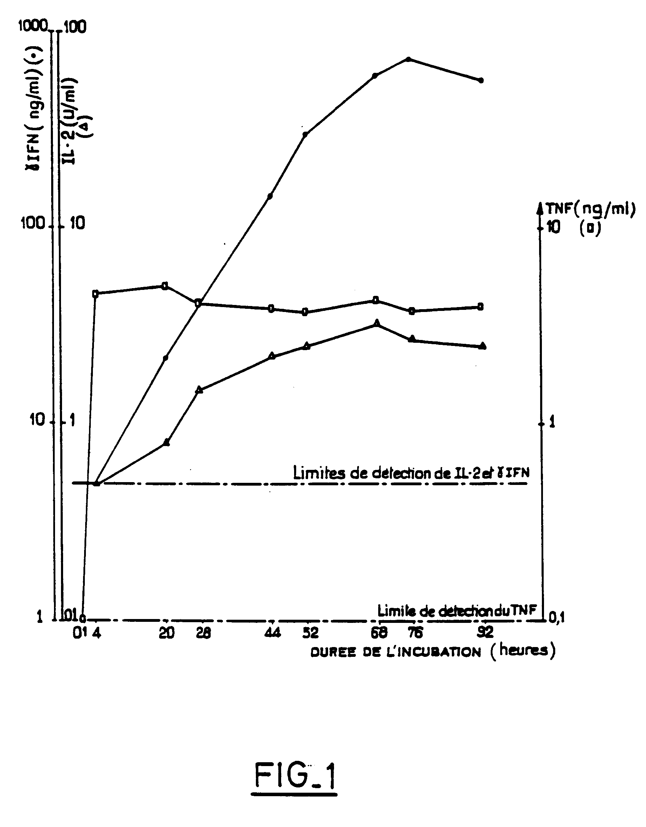

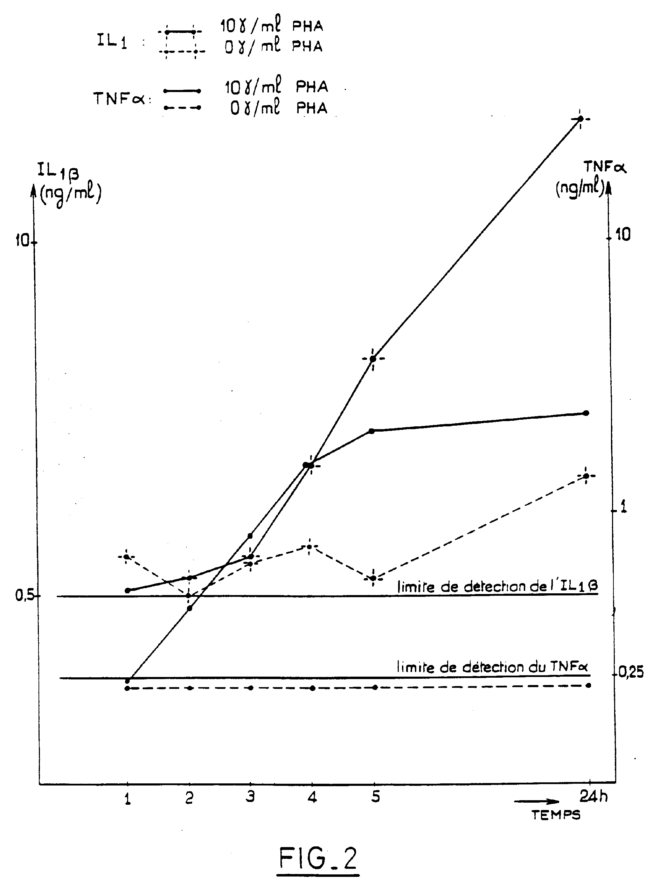

A l'origine de la présente invention, on a découvert que les monokines, comme le TNFα ou l'IL-1β, sont sécrétées beaucoup plus rapidement que les autres cytokines dans les milieux de culture de cellules (voir figures 1 et 2), à tel point que de façon surprenante et originale, l'étape de culture des cellules mononucléés du sang périphérique pouvait être évitée pour le dosage des monokines en dosant directement le plasma pourvu que celui-ci soit obtenu à partir du sang total, stimulé à l'aide d'un agent mitogène ayant toute étape de séparation entre plasma (ou sérum) et cellules sanguines.

La présente invention a donc pour objet un dosage de monokines d'un échantillon sanguin, notamment de TNFα ou d'IL-1β, caractérisé en ce que l'on dose la monokine directement dans le sérum ou dans le plasma, celui-ci étant obtenu à partir du sang total, préalablement stimulé par un agent mitogène avant séparation du plasma ou sérum.

Dans un mode de réalisation particulier, l'échantillon de sang à doser est prélevé sur un anticoagulant, par exemple de l'EDTA, puis mis en contact avec l'agent mitogène, par exemple de la PHA et incubé tel quel. A l'issue de cette opération, le sang est décanté par centrifugation, la monokine étant dosée dans le plasma obtenu.

La stimulation du sang total peut se faire notamment avec 0,02 à 50 μg/ml de phytohémagglutinine (PHA) ou 10 à 200 μg/ml de Lipopolysaccharide (LPS) pour des incubations de 30 minutes à 48 heures, à des températures pouvant être comprises entre 4°C à 40°C.

Lorsque la température ou la durée d'incubation diminue, la stimulation requiert une concentration en agent mitogène plus importante.

Pratiquement, il est approprié de réaliser une stimulation avec par exemple 0,1 à 10 μg/ml de PHA ou 15 à 75 μg/ml de LPS pour des durées d'incubation de 2 à 4 heures à température de 20 à 37°C.

En particulier, on peut effectuer une stimulation directe du sang total avec par exemple 5 μg/ml de PHA et 25 μg/ml de LPS pour des incubations de 2 heures à 37°C.

On obtient ainsi des valeurs de concentrations plasmatiques du TNFα en dosage aussi bien RIA que IRMA de l'ordre de $10^3$ pg/ml avec des variations significatives de sujet à sujet, c'est-à-dire des valeurs exploitables qui satisfont aux conditions mentionnées précédemment.

Cette méthode de dosage de monokines par stimulation directe du sang total sans recours à une étape de culture présente en outre une série d'avantages qui facilitent grandement leur application en routine clinique en ce qu'elle est notamment :
— rapide (2 heures d'incubation pour la stimulation du sang total, moins de 2 heures d'incubation pour le dosage en cas de dosage immunologique IRMA, comme tout échantillon plasmatique),
— simple, puisqu'elle ne demande pas de conditions de stérilité, ni d'une infrastructure de culture,
— utilisable sur les lieux de prélèvements sanguins, équipés d'un incubateur simple,
— fiable, puisque reproductible de patient à patient et de prélèvement à prélèvement, puisqu'elle élimine toutes les variations qu'entraînent les cultures.

De plus, on peut fournir, dans les trousses de dosages, des tubes de prélèvement sanguin contenant déjà l'agent mitogène, comme il sera développé plus loin, sinon, on dispose d'un délai de deux heures pour démarrer la stimulation après le prélèvement. Par conséquent, l'incubateur peut se situer dans un rayon de deux heures du lieu de prélèvement.

Outre la phytohémagglutinine (PHA) ou les lipopolysaccharides (LPS), on peut également citer comme agent mitogène, par exemple l'acétate de phorbol myristate, l'ionophore de calcium, le zymosan, la digitonine, la WGA (Wheat Germ Agglutinine) ou d'autres lectines, comme la concanavaline.

Ci-après sont indiquées à titre illustratif des gammes appropriées de concentrations pour l'utilisation de ces différents agents mitogènes.
1) TPA = MPA (phorbol myristate acétate) (PM = 617)
    $10^{-7}$ à $10^{-9}$ M
2) Calcium ionophore (PM = 523)
soit le composé A 23187 (Sigma) : 50 à 100 ng/ml,
soit ionomycine (Calbiochem) = 0,5 à 2 μM.
3) Zymosan A :
    300 μg/ml à 3 mg/ml.
4) Digitonine :
    1 à 20 μg/ml.
5) WGA (Wheat Germ Agglutinin) : 10 à 100 μg/ml.

6) Con A (Concanavalin A) :

2 à 10 µg/ml.

On peut citer comme anticoagulant selon l'invention outre l'EDTA, l'héparine ou encore le citrate.

Les dosages selon l'invention sont des dosages immunologiques, tels que les dosages radioimmunologiques. Notamment, il peut s'agir de dosages immunologiques par déplacement tels que des dosages RIA ou des dosages immunométriques à deux sites notamment du type IRMA.

Les dosages par déplacement sont basés sur une compétition entre l'antigène natif à doser et l'antigène marqué, vis-à-vis de l'anticorps. Après réaction, le complexe antigène-anticorps est séparé de l'antigène libre. Généralement, la mesure est effectuée sur l'antigène lié. Dans ces conditions et en présence d'une concentration constante en anticorps, le signal mesuré diminue lorsque la concentration de l'antigène al doser augmente.

Dans le cas de dosages immunométriques à deux sites, il s'agit de doser un antigène avec :
– soit deux anticorps différents et complémentaires qui se fixent sur deux épitopes différents,
– soit deux anticorps identiques qui se fixent sur un épitope répétitif.

Le premier anticorps est immobilisé sur une phase insoluble (cellulose, surface plastic, Magnogel, etc.) et le deuxième non adsorbé porte un traceur ou un marqueur, il est dit marqué.

Après réaction, la séparation du complexe libre-lié est réalisée par simple lavage et le complexe lié est mesuré. Dans ces conditions, et en présence d'un excès d'anticorps, le signal mesuré augmente lorsque la concentration de l'antigène à doser augmente.

Dans ces deux types de dosages, le traceur ou marqueur permet de quantifier la réaction immunologique. Il est fixé soit sur l'antigène dans le cas d'un dosage par déplacement, soit sur l'anticorps pour un dosage immunométrique. Quel que soit le type de dosage, le choix du marqueur dépend à la fois de la sensibilité désirée et des équipements des laboratoires d'analyses. Actuellement, il existe quatre classes principales de traceurs :
– les isotopes radioactifs. L'iode 125 représente le meilleur compromis entre la sensibilité et la stabilité. Par déplacement, les dosages sont de type RIA (Radio Immuno Assay), et par immunométrie, les dosages sont de type IRMA (Immuno Radio Metric Assay) ;
– les enzymes telles que la $\beta$-galactosidase, la phosphatase alcaline ou la peroxydase. Par déplacement, les dosages sont de type ELISA (Enzyme Linked Immuno Sorbent Assay), et par immunométrie, les dosages sont de type IEMA (Immuno Enzymometric Assay).

Enfin, la présente invention a également et naturellement pour objet un kit de dosage ou trousse de réactifs pour dosages immunologiques de monokines comportant, entre autres réactifs, un échantillon d'agent mitogène tel que de la PHA provenant d'un lot parfaitement calibré.

Par exemple, la trousse comportera un tube de prélèvement sous vide, tel qu'un vacutainer pouvant prélever 5 à 10 ml de plasma, comportant de l'EDTA et de la PHA ou uniquement de l'EDTA. Dans ce dernier cas, l'échantillon à doser recueilli dans le tube contenant de l'EDTA sera ensuite transvasé dans un tube contenant de la PHA.

La livraison d'une trousse de dosages comportant l'agent mitogène contribue à la fiabilité du dosage dans la mesure où ces substances mitogènes sont des lectines complexes et donc difficilement reproductibles de lot à lot, les variations dans la composition pouvant entraîner des différences d'activités dans la stimulation.

D'autres caractéristiques et avantages de la présente invention apparaîtront à la lumière de la description qui va suivre faite en référence aux figures suivantes.

. la figure 1 présente les cinétiques de libération du TNF$\alpha$ ($\square$) de l'IL-2 ($\triangle$) et du $\gamma$IFN ($\bullet$) dans des cultures de cellules mononucléées stimulées par PHA (1 µg/ml pour $\gamma$IFN et 10 µg/ml pour IL-2 et TNF).

Cette figure établit la rapidité de la sécrétion du TNF$\alpha$ dans les milieux de culture stimulés.

. la figure 2 représente les cinétiques de libérations entre 0 et 6 heures du TNF$\alpha$ et de l'Il-1$\beta$ respectivement dans des cultures de cellules mononucléées stimulées par de la PHA ( 10 µg/ml).

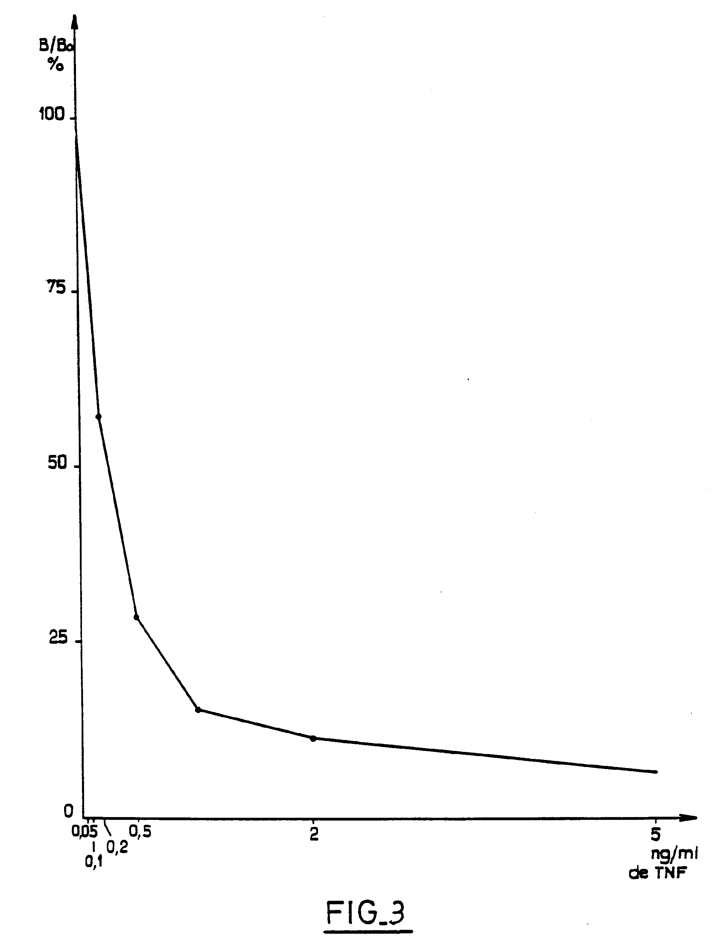

. la figure 3 représente une courbe étalon RIA du TNF$\alpha$ .

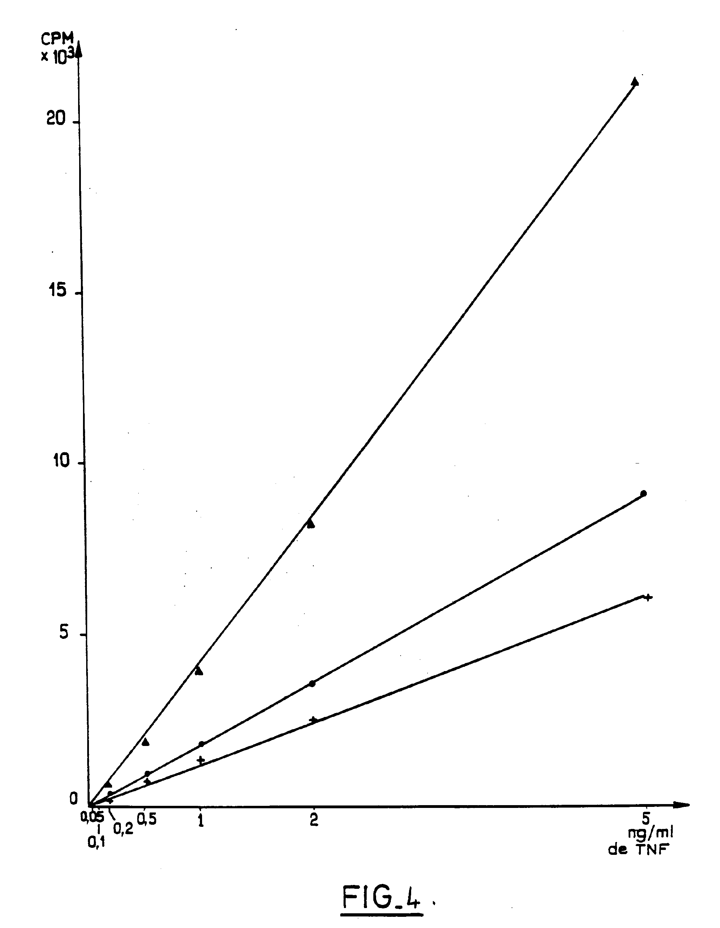

. les figures 4 et 6 représentent des courbes standard IRMA du TNF$\alpha$ avec divers paires d'anticorps monoclonaux (ACM adsorbé/ACM marqué).

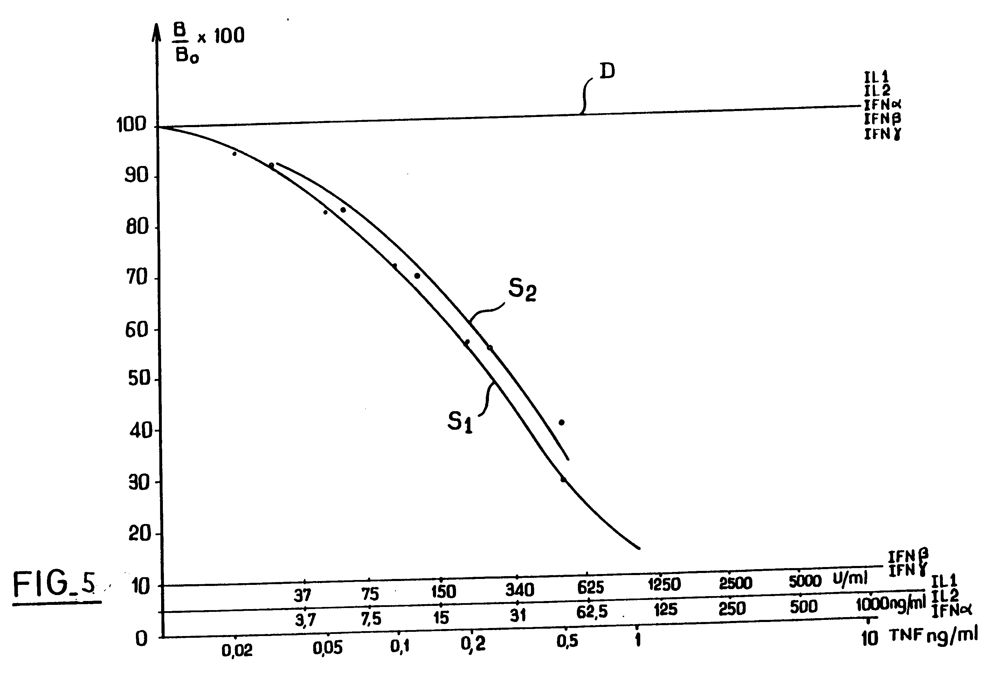

. la figure 5 représente la courbe semi-logarithmique de spécificité des dosages RIA pour le TNF$\alpha$.

. la figure 7 représente une courbe de corrélation entre dosages IRMA et RIA du TNF$\alpha$.

EXEMPLE I - Culture de cellules mononucléés

Généralement utilisée pour étudier l'incorporation de thymidine tritiée (J. Exp. Med., 1979, 149, 1029), cette méthode de culture a été en fait repensée quant à deux paramètres pour son application à l'étude de la libération de TNF$\alpha$ : la durée de culture et les doses optimales de PHA.

La méthodologie de ce type de culture est classique, cependant, il y a été apporté deux modifications.

La première modification apportée aux cultures classiques a été l'usage de LeucoPrep® (Becton Dickinson). Ce produit permet une séparation simplifiée et plus rapide des cellules mononucléés du sang périphérique, puisqu'il ne demande pas de traitement ni de dilution préalable du sang.

La deuxième modification a été:un dosage d'ADN effectué à l'issue des cultures sur le culot cellulaire. Il est, en effet, utile de ramener les taux de TNF détectés par le RIA dans le milieu de culture, à un même nombre de cellules, le mitogène (PHA) utilisé pouvant montrer des effets variables d'un échantillon à l'autre.

## 1. Isolement des cellules

Le sang d'un sujet "normal" est prélevé sur EDTA par tubes Vacutainer et déposé, pendant 6 heures où il est maintenu à température ordinaire, dans des tubes contenant les deux phases de LeucoPrep (Becton Dickinson). Ces tubes sont centrifugés à 900 g (20°C/15 min.). A l'issue de cette séparation, les cellules mononucléés forment un anneau blanc visible entre le plasma et la phase organique. La couche cellulaire est prélevée stérilement et déposée dans un tube à fond conique de 50 ml, où les cellules sont lavées deux fois consécutivement par 30 ml de tampon PBS contenant 0,1 % $NaN_3$ (300 g/ 4°C/ 10 min. et 400 g/ 4°C/ 10 min.). Les cellules sont reprises par 3 ml de milieu de culture RPMI 1640 contenant glutamine, antibiotiques et 5 % de sérum autologue. Les cellules sont comptées et la concentration cellulaire, ajustée à $10^6$ cell/ml.

## 2. Cultures et stimulation par mitogène

Les cultures sont effectuée dans des plaques 96 puits NUNC où 200 μl de la suspension cellulaire sont déposés par puits, auxquels s'ajoutent 22 μl d'une solution de phytohémagglutinine PHA (Wellcome).

Les cultures sont incubées à 37°C (5% $CO_2$ et 95% air).

Les surnageants sont préleves apres centrifugation des boites 96 puits (900 g/10 min/20°C) et conservés à -20°C pour dosages radioimmunologiques. Les cellules -adhérant au fond des puits- sont reprises par 200 μl de $H_2O$ distillées et traitées deux secondes par ultrasons. Une prise aliquote de 150 μl de contenu cellulaire est'dosée par la méthode de Paigen et Labarca pour apprécier leur concentration en DNA.

Tous les résultats doivent être rapportés à l'unité de concentration de DNA.

## 3. Résultats

Deux études préliminaires ont été réalisées : une cinétique et une étude dose-réponse.

Les concentrations finales de PHA étudiées pour l'effet dose-réponse varient de 0,02 à 50 μg/ml.

Les cinétiques ont été effectuées jusqu'à 4 jours d'incubation.

### a. Effet dose-réponse de PHA.

Le γ-IFN montre des courbes réponses en forme de cloche avec maximum pour des doses de 1 et 2 μg/ml de PHA.

D'autre part, le TNFα n'est pas stimulé par rapport au témoin par des doses inférieures à 1 μg/ml ; au-delà de cette valeur, la progression est dose-dépendante.

Enfin, l'IL-2 ne montre une stimulation décelable que dans une gamme étroite de concentration - 2 à 20 μg/ml - avec un maximum à 5 μg/ml.

Conclusion : des doses de 1 et de 10 μg/ml de PHA semblent les plus appropriés pour les trois paramètres γ-IFN, IL-2, TNFα.

### b. cinétique.

La stimulation du γ-IFN par 1 et 10 μg/ml montre des cinétiques semblables, croissantes jusqu'à trois jours de culture pour plafonner dans les heures qui suivent.

L'IL-2 est libérée de façon différente suivant les doses de PHA : par 1 μg/ml, il y a un maximum à 28 h entre 20 et 44 h), puis une décroissance jusqu'à arriver, au bout de 76 h, à des taux inférieurs à la limite de détection du dosage. Par 10 μg/ml par contre, la libération s'effectue progressivement jusqu'al 52 h, après quoi elle présente un palier.

Par contre, le TNFα et l'IL-1β sont stimulés très tôt dans la culture (moins de 4 h) à des taux qu'il conservera dans la suite et qui sont dose-dépendants du PHA comme il apparaît sur la figure 1 et la figure 2.

### 4. Culture pour le TNFα et l'IL-1β

a. durée de culture.

Une cinétique de culture, stimulée respectivement par 0, 1, 10 et 50 µg/ml d'un lot de PHA (Wellcome), nous a permis de connaître la loi de libération de TNFα et IL-1β en fonction du temps, chez les sujets sains.

Résultats :

Pour les 4 doses de PHA, les taux de TNFα et IL-1β dans le milieu de culture croissent jusqu'à 4 h de culture, mais à partir de ce moment, ils restent constants jusqu'à plus de 90 h de culture pour le TNFα et continuent à croître pour l'IL-1β.

Conclusion :

Pour l'étude du TNFα et de l'IL-1β, les cellules en culture doivent être incubées moins de 4 heures avec la PHA ; les durées plus longues ne montrant pas de modifications sensibles dans la libération de cette molécule.

b. doses de PHA à utiliser.

Une étude dose-réponse a été effectuée par des doses variables de PHA (de 20 ng à 50 µg/ml) pendant 72 h de culture.

Résultats :

jusqu'à 500 ng/ml de PHA, les modifications des taux de TNFα sont indécelables puisque inférieurs à la limite de détection du dosage. Au-delà de 500 ng/ml, par contre, il existe une relation pratiquement linéaire, dose-dépendante et ce, jusqu'al 50 µg/ml de PHA.

Conclusion :

Les doses importantes de PHA permettent la libération maximale de TNFα alors que les autres paramètres (γ-IFN, IL-2) sont diminués suite vraisemblablement à un effet toxique de la PHA.
En pratique, des doses de 1, 5 et 10 µg/ml du mitogène permettent d'apprécier convenablement le potentiel d'une cellule à libérer son TNFα.

### EXAMPLE II : Stimulations directes dans le sang

Sur la base des temps relativement courts mis en évidence plus haut, une méthode originale a été développée pour court-circuiter la partie "culture". En bref, le sang du donneur, prélevé sur EDTA, est immédiatement mis en contact de la PHA et incubé tel quel. A l'issue de cette opération, le sang est décanté par centrifugation. Le TNFα est alors dosé directement dans le plasma obtenu.
Le sang de 9 sujets "normaux" est prélevé sur EDTA et immédiatement soumis à une stimulation par 0, 1 et 10 µg/ml de PHA. Après par exemple 4 h d'incubation à 37°C, les tubes sont décantés et les plasmas dosés en TNFα.
Comme pour les cultures de cellules mononucléées, le TNFα est aussi stimulé de manière dose-dépendante. Ainsi, par rapport à une moyenne des témoins (0 µg/ml de PHA) de 53,8 ± 10,5 U/ml, 1 µg/ml donne des taux plasmatiques moyens de 512 ± 273 et 10 µg/ml, 1966 ± 804 pg/ml.

Choix des conditions de température et de durée d'incubation :

Des stimulations directes par 5 µg/ml de PHA ont été effectuées sur le sang de 4 sujets "normaux" pour des durées de 1/2 h, 1h, 2h, 6h, 22h à 20°C et à 37°C.

Résultats :

1. A température ambiante (20°C), les stimulations sont toujours beaucoup plus faibles qu'à 37°C.

2. Pour les 4 sujets, les taux plasmatiques de TNFα les plus importants sont obtenus au bout de 2 h d'incubation et restent ensuite stable en plateau.

Conclusion :

Les stimulations directes par 5 μg/ml de PHA montrent les meilleurs résultats pour des incubations de 2 h à 37°C. La séparation du plasma après stimulation directe du sang peut être pratiquée après un délai de 0 heures.

Intérêt de la stimulation directe :

Cette dernière méthode possède l'avantage substantiel d'être comme on l'a vu :
1) rapide (2 h) et
2) simple puisqu'elle ne demande pas de conditions de stérilité, ni d'une infractructure de culture.
Elle est donc utilisable sur les lieux de prélèvements sanguins équipés d'un incubateur simple et d'une centrifugeuse, l'échantillon étant ensuite envoyé au laboratoire de dosage où le plasma sera séparé comme pour tout échantillon sanguin prélevé sur anticoagulant pour dosage plasmatique de paramètre biologique de routine.

EXEMPLE III : Dosage du type RIA du TNFα

1. Immunisation

Deux lapins ont reçu une injection intradermale d'une émulsion de 0,5 ml d'une solution saline contenant 50 μg de TNFα (TNFα humain recombinant) et 0,5 ml d'adjuvant de FREUND selon la méthode VAITUKAITIS (6).
Quatre injections de rappel mensuelles ont été ensuite administrées en utilisant les mêmes quantités d'antigène. Des échantillons de sang ont été régulièrement recueillis à partir de la veine marginale de l'oreille dans chaque lapin pour tester le titre d'antisérum.

2. Marquage du TNFα

Le TNFα est marqué avec de l'iode 125 en utilisant de la chloramine T selon la méthode de GREENWOOD ( 7). 5 μg de TNFα, dissous dans 5 μl de tampon phosphate 0,05 M à pH 7,5, sont incubés avec 1 mCi de I$^{125}$, 5 μl du même tampon phosphate 0,5 M - pH 7,5 et 20 μg de chloramine T dissoute dans 10 μl du tampon phosphate 0,05 M - pH 7,5.
Après 30 secondes, on arrête la réaction par addition de 20 μg de métabisulfite dilué dans 10 μl du même tampon. Après chromatographie par perméation sur gel avec une colonne Séphadex G50® (0,9 x 30 cm), les fractions correspondant au premier pic radioactif sont passées sur colonne Séphadex G100® (0,9 x 65 cm). L'éluant pour les deux colonnes est le tampon phosphate 0,05 M - pH 7,5, contenant 0,05 % de NaN$_3$, 0,1 % d'albumine de bovin.

3. Méthode de dosage et courbe étalon

Des incubations sont effectuées à température ambiante dans le tampon phosphate 0,05 M - pH 7,5, contenant 0,5 % de BSA, 0,05 % de NAN$_3$, ; 0,1 ml de tampon contenant des quantités croissantes d'antigène non marqué (5, 10, 20, 50, 100, 200, 500 pg) ou 0,1 ml de l'échantillon à doser sont mélangés avec 0,1 ml d'antisérum à une dilution de 1/125 000 ; après une nuit de préincubation, 0,1 ml de tampon d'incubation contenant de l'antigène marqué correspondant environ à 30.000 cpm et du sérum de lapin à dilution de 1 % sont ajoutés ; l'incubation se fait à température ambiante pendant 20 à 24 heures. Puis l'anticorps lié à l'antigène est précipité par addition de 1 ml d'un tampon d'incubation contenant 0,5 % de Tween®, 6 % de polyéthylène glycol, 0,2 % de cellulose microcrystalline et 0,5 % de sérum de globuline anti- lapin préparé chez le mouton. Après 20minutes d'incubation à température ambiante, le complexe antigène-anticorps est sédimenté par centrifugation pendant 20 minutes à 2.500 g. Le surnageant est analysé au compteur de radioactivité γ.
La figure 3 représente la courbe d'étalonnage RIA obtenue pour une incubation totale de deux jours à température ambiante comme suit :

```
.  antisérum + standard ou échantillon    :  1 jour

.  + TNF - I¹²⁵                            :  1 jour

.  + immunoprécipitant                     :  20 minutes
```

et après centrifugation et comptage.

On appelle conventionnellement B la radioactivité du précipité (celle de l'antigène marqué lié à l'anticorps). On connaît aussi la radioactivité totale initiale, qui est la même pour tous les tubes que l'on appelle T. On peut aussi estimer la radioactivité de l'antigène marqué libre (appelée Bo). On se contente généralement d'estimer Bo = T-B.

4. Spécificité

De l'IFN $\beta$ et $\gamma$ recombinants dans des concentrations de 0 à 5000 U/ml, de l'IL-1, IL-2 et IFN$\gamma$ recombinants dans des quantités de 1000 ng/ml ont été incapables de produire une inhibition de la liaison du TNF$\alpha$ marqué à l'antisérum et n'interfèrent donc en rien dans le dosage du TNF$\alpha$ comme le montre la droite D sur la figure 5.

Par ailleurs, sur cette figure 5, la courbe $S_1$ représente la courbe de déplacement pour du TNF standard froid. La courbe $S_2$ de droite représente une même courbe pour différentes dilutions d'un milieu de cultures possédant du TNF endogène.

Le parallélisme de ces deux courbes confirme le comportement immunologique identique entre le standard et le milieu de culture et confirme donc qu'on peut avoir recours à une stimulation.

EXEMPLE IV : Dosage du type IRMA du TNF$\alpha$ - METHODES

1) Immunisation

Deux souris femelles âgées de deux mois, de souche BALB /c, provenant de CEN-Mol, ont été immunisées avec du TNF recombinant selon la procédure suivante :
– une première injection intrapéritonéale de 10 $\mu$g de TNF dans un adjuvant complet de FREUND à 50 % au jour 0,
– une seconde injection intrapéritonéale de 10 $\mu$g de TNF dans un adjuvant de FREUND incomplet à 50 % au jour 20,
– une troisième injection intrapéritonéale de 10 $\mu$g de TNF dans du PBS au jour 50,
– une des deux souris a reçu une injection de rappel de 10 $\mu$g de TNF$\alpha$ dans du PBS intrapéritonéalement au jour 75 et la deuxième souris après 4 mois.

2) Fusion

Les deux souris ont été tuées trois jours après le dernier rappel. Les rates ont été prélevées et les cellules de rate ont été préparées pour une fusion.

La fusion de cellules a été effectuée suivant le protocole de KHOLER et MILSTEIN (8) modifié. $9.10^7$ cellules de rate ont été fusionnées avec $1,6.10^7$ cellules de myélome SP2-O pour la première souris et $4,8.10^7$ cellules de rate ont été fusionnées avec $10^7$ cellules de myélome SP2-O pour la deuxième souris, en utilisant comme agent fusogène le polyéthylène glycol 4000. Après fusion, les cellules ont été remises en suspension à la concentration de $2,5.10^5$ cellules/ml dans un milieu de cultures DMEM complémenté par 10% de sérum de cheval, 10 % de sérum de veau foetal, 4 $\mu$g/l d'insuline de porc, 2 % de solutions GIBCO de pénicilline-streptomycine, glutamine, amino-acide non essentiels, pyruvate de sodium, hypoxanthine-thymidine et aminoptérine. La solution a été distribuée dans 20 plaques de 96 puits avec 0,2 ml/puits.

3) Tri des hybridomes

10 jours après la fusion, le milieu de cultures des plaques a été prélevé et remplacé par le même milieu sans aminoptérine, ni insuline. Les plaques ont été triées avec un microscope à inversion pour détecter les puits avec hybridomes en croissance. La présence d'anticorps spécifiques du TNF dans le milieu de cultures des puits à hybridomes positifs a été sélectionnée comme suit : 50 ml du milieu ont été prélevés des puits et incubés une nuit avec 40.000 cmp de TNF -[125] I dans 0,2 ml de tampon PBS dans des tubes à hémolyse. 0,1 ml de réactif Wellcome anti-souris d'âne couplé à de la cellulose ont été ajoutés dans chaque tube pendant 15 minutes. 2 ml d'eau contenant 0,1 % de Tween 20 ont été ajouté aux tubes avant centrifugation à 4000 cpm pendant 10 minutes. Le surnageant a été prélevé et la radioactivité comptée par un compteur $\gamma$.

### 4) Production d'anticorps monoclonaux

Les cellules des puits positifs ont été transférées successivement dans des plaques 24 puits et des boites de pétri.

$5.10^6$ cellules par souris ont été ensuite injectées intrapéritonéalement dans des souris BALB/c, ayant subi une injection 10 jours avant de 0,5 ml de pristane. 10 à 15 jours après, les souris ont produits un fluide ascitique, contenant des anticorps monoclonaux. Ces fluides ascitiques ont été recueillis et purifiés.

### 5) Purification des anticorps monoclonaux

Les anticorps monoclonaux du fluide ascitique ont été purifiés à travers une colonne d'affinité protéine-A-sépharose pharmacia.

### Dosage immunoradiométrique

100 tubes maxisorbe Nunc ont été revêtus à l'aide de 200 $\mu$l d'une solution à 20 ng/ml de chaque anticorps monoclonal. Après 48 heures de revêtement, la solution d'anticorps a été retirée et les tubes ont été saturés avec du tampon phosphate contenant 0,5 % de BSA pendant 4 heures. Ensuite, les tubes ont été lavés deux fois avec 2 ml d'eau contenant 0,1 % de Tween 20®. Les tubes ont été séchés par aspiration et préservés dans un emballage hermétique avec un déshydratant.

25 $\mu$g de chaque anticorps monoclonal ont été marqués avec du Na125I par une technique à la chloramine-T.

Chaque anticorps monoclonal marqué à l'iode 125 a été utilisé dans un dosage immunoradiométrique avec chacun des anticorps monoclonaux adsorbés sur tubes maxisorb.

0,005 ou 5 nanogrammes/ml de standards TNF dans 0,1 ml de tampon phosphate pH 7 contenant 0,5 % de BSA ont été incubés avec 200 000 cpm d'anticorps monoclonaux marqués à $^{125}$I dans 50 microlitres de tampon phosphate pH 6 et cela pendant deux heures dans des tubes revêtus des différents anticorps monoclonaux. Le mélange a été ensuite prélevé et les tubes ont été lavés deux fois avec de l'eau contenant 0,5 % de Tween 20 et comptés avec un compteur $\gamma$.

Les couples d'anticorps monoclonaux donnant le meilleur signal pour les concentrations en TNF$\alpha$ données ont été retenus pour la suite.

### EXEMPLE V - Dosage plasmatique IRMA du TNF$\alpha$ avec activation

### - RESULTATS -

### 1) Production des hybridomes

Des cellules de rate de souris de souche Balb/c ont été fusionnées avec des cellules de myélome SP2-0. 1380 surnageants des puits contenant au moins un clone(hybridome)ont été analysés quant à la présence d'anticorps reconnaissant le TNF$_\alpha$.

19 hybridomes secrétant des anticorps spécifiques anti-TNF$\alpha$ ont été sélectionnés. Ces hybridomes sont les suivants : 3C5, 1B5, 1D6, 3B5, 1B2, 4B3, 5C1, 2C4, 5A3, 2B3, 6A3, 4D1, 2A5, 3C3 , 3E6, 3D3, 1F9, 2H7 et 10A2.

Le tableau I ci-après décrit les caractéristiques de liaison du TNF$\alpha$ marqué à l'iode 125 avec les anticorps monoclonaux sécrétés par 5 de ces clones : 3D3, 1F9, 2H7, 3E6,10A2 qui étaient les seuls dont on disposait initialement.

## Tableau I

| Anticorps | % de fixation I$^{125}$-TNF (*) |
|---|---|
| 3D3 | 61 |
| 1F9 | 60 |
| 2H7 | 59 |
| 10A2 | 15 |
| 3E6 | 11 |

(*) fixation de I$^{125}$-TNF de surnageant de plaques 24 puits.

2) Dosage immunoradiométrique

La culture des cinq hybridomes a été développée et les cellules ont été injectées dans des souris de souche Balb/c pour obtenir un fluide ascitique. Les anticorps ont été purifiés à partir du fluide ascitique sur une colonne de protéine-A. Des anticorps purifiés ont été revêtus sur des tubes maxisorbes et marqués avec du Na125I. Toutes les combinaisons de chaque anticorps revêtu avec chaque anticorps marqué ont été essayées dans un dosage immunoradiométrique avec trois standards.

Les résultats figurent dans le Tableau II ci-après. Les valeurs données sont en cpm de TNF-125I-fixé.

## TABLEAU II

| ACM adsorbés | | 3D3 | 1F9 | 2H7 | 10A2 |
|---|---|---|---|---|---|
| ACM marqués | standards ng/ml | | | | |
| 3D3 | 0 | 366 | 379 | 433 | 479 |
| | 0.5 | 1055 | 1395 | 1728 | 1142 |
| | 5 | 4402 | 7955 | 10093 | 7935 |
| 1F9 | 0 | 752 | - | - | 1276 |
| | 0.5 | 1330 | - | - | 1150 |
| | 5 | 6257 | - | - | 3374 |
| 2H7 | 0 | - | - | - | - |
| | 0.5 | - | - | - | - |
| | 5 | - | - | - | - |
| 10A2 | 0 | - | - | - | - |
| | 0.5 | - | - | - | - |
| | 5 | - | - | - | - |

Le Tableau II montre que le couple anticorps 2H7 adsorbé avec anticorps 3D3 marqué donne le meilleur signal pour chaque concentration en TNF. Ces conclusions sont confirmées par une seconde expérience effectuée avec une plus large échelle de standards, comme le montre la figure 3.

La figure 3 présente des courbes standards IRMA avec une incubation totale de deux heures à température ambiante :

. anticorps 1 adsorbé + standards ou échantillons + anticorps 2 marqué à l'iode 125 ont été incubés ensemble pendant deux heures.

Trois courbes ont été réalisées respectivement pour les couples 2H7 adsorbé /3D3 marqué (▲), 1F9 adsorbé /3D3 marqué (●), 3D3 adsorbé / 1F9 marqué (+).

Il ressort du Tableau II que l'anticorps 3D3 donne une réponse significative en couplage avec lui-même marqué. Ce paradoxe peut être expliqué par le fait que le TNF peut exister aussi bien sous forme monomérique que sous forme bi- ou tri-mérique. Dans ces deux derniers,cas, un même épitope peut être disponible pour l'anticorps adsorbé et l'anticorps marqué.

3) Spécificité de l'IRMA

La spécificité du dosage a été vérifiée avec six réactifs potentiels pouvant donner des réactions croisées : le TNFα, l'interleukin-1, l'interleukin-2, l'interféron α, l'interféron y et l'interféron β. L'expérience a été effectuée en remplaçant les standards TNFα par des standards de réactifs croisés, concentrations plus importantes. Ces concentrations étaient

```
TNFα      :  0 à 50 ng/ml
TNFβ      :  0 à 1000 ng/ml
IL-1      :  0 à 1000 ng/ml
IL-2      :  0 à 5000 U/ml
IFNα      :  0 à 1000 ng/ml
IFNγ      :  0 à 5000 U/ml
IFNβ      :  0 à 5000 U/ml.
```

Aucun de ces six réactifs potentiels quant à des réactions croisées, n'a donné lieu à une interférence affectant le titre en TNFα. En conséquence, le dosage est complètement spécifique pour le TNFα pour les concentrations données de réactifs. Le dosage IRMA proposé pour le TNFα est donc spécifique par rapport au TNFβ, l'interleukin-1, l'interleukin-2, l'interféron α, l'interféron γ, l'interféron β.

4) Evaluation clinique

Le couple d'anticorps monoclonaux 2H7 adsorbé avec 3D3 marqué a été choisi pour les expériences suivantes.

Le dosage IRMA a été comparé avec le RIA dans différentes séries d'échantillons cliniques.

La première série consistait dans des surnageants de cellules mononuclées normales activés ou non avec de la PHA. La comparaison des titres en TNFα par RIA et IRMA pour ces échantillons est donnée au Tableau III ci-après.

La seconde série consiste dans des sérums de donneurs normaux après stimulation directe du sang total par 0,1 ou 10 μg de PHA. Les résultats comparatifs sont donnés au Tableau IV.

Au Tableau IV, les valeurs sont données en Pg/ml de TNFα.

A désigne l'expérience sans stimulation par PHA,

B désigne l'expérience avec stimulation par 1 μg/ml de PHA,

C désigne l'expérience avec stimulation par 10 μg/ml de PHA.

Tous les IRMA ont été éxécutés en deux heures à température ambiante en une étape.

Tableau III

| Surnageant | RIA polyclonal TNFα valeurs (ng/ml) | IRMA monoclonal TNFα valeurs (ng/ml) |
|---|---|---|
| 1 | 1,2 | 1,2 |
| 2 | 0,55 | 0,51 |
| 3 | 0,86 | 1,00 |
| 4 | 0,34 | — |
| 5 | 0,52 | 0,65 |
| 6 | 2,8 | 3,1 |
| 7 | 0,48 | 0,87 |
| 8 | 0,51 | 0,56 |
| 9 | 0,80 | 0,36 |
| 10 | 1,00 | 0,95 |

## Tableau IV

| Echantillon | RIA Polyclonal TNFα - Valeurs (pg/ml) | IRMA monoclonal TNF α Valeurs (pg/ml) |
|---|---|---|
| 1-A | 70 | – |
| 1-B | 780 | 760 |
| 1-C | 3000 | 2440 |
| 2-A | 48 | – |
| 2-B | 1050 | 220 |
| 2-C | 2800 | 2000 |
| 3-A | 68 | – |
| 3-B | 194 | – |
| 3-C | 950 | 660 |
| 4-A | 36 | – |
| 4-B | 240 | – |
| 4-C | 1090 | 250 |
| 5-A | 52 | – |
| 5-B | 360 | 150 |
| 5-C | 830 | 650 |
| 6-A | 50 | – |
| 6-B | 520 | – |
| 6-C | 1720 | 1100 |
| 7-A | 50 | – |
| 7-B | 545 | 400 |
| 7-C | 2550 | 2000 |
| 8-A | 52 | – |
| 8-B | 460 | 240 |

Le tableau V ci-après présente pour une troisième série de 20 dosages de sera de sujets sains sans stimulation, des résultats comparatifs RIA et IRMA. (Ce TNFα en dosage IRMA n'était pas détectable avec le couple 2H7/3D3).

Le tableau VI présente une série de 20 dosages de sera de sujets atteints de pancréatite sans stimulation.

Tableau V

| Sérum | RIA TNFα (pg/ml) | IRMA TNFα (pg/ml) |
|---|---|---|
| 1 | 42 | − |
| 2 | 44 | − |
| 3 | 53 | − |
| 4 | 62 | − |
| 5 | 23 | − |
| 6 | 31 | − |
| 7 | 39 | − |
| 8 | 77 | − |
| 9 | 58 | − |
| 10 | 36 | − |
| 11 | 34 | − |
| 12 | 45 | − |
| 13 | 74 | − |
| 14 | 59 | − |
| 15 | 29 | − |
| 16 | 28 | − |
| 17 | 47 | − |
| 18 | 47 | − |
| 19 | 69 | − |
| 20 | 51 | − |

## Tableau VI

| Sérum | RIA TNFα (pg/ml) | IRMA TNFα (pg/ml) |
|---|---|---|
| 1 | 183 | - |
| 2 | 142 | - |
| 3 | 56 | - |
| 4 | 184 | - |
| 5 | 145 | - |
| 6 | 165 | - |
| 7 | 179 | - |
| 8 | 196 | - |
| 9 | 105 | - |
| 10 | 157 | - |
| 11 | 162 | - |
| 12 | 197 | - |
| 13 | 134 | - |
| 14 | 141 | - |
| 15 | 167 | - |
| 16 | 109 | - |
| 17 | 125 | - |
| 18 | 112 | - |
| 19 | 115 | - |
| 20 | 134 | - |

En conclusion, le titrage en TNFα de sérum d'échantillons de sang activés par PHA ou de surnageants de monocytes activés par PHA donne des valeurs importantes qui sont satisfaisantes en RIA et IRMA, au regard de la sensibilité de ces dosages. En revanche, en l'absence de stimulation, on observe un nivellement des valeurs, celles-ci étant de surcroît trop faibles par rapport à la limite de détection des dosages.

REFERENCES BIBLIOGRAPHIQUES

(1) J. SAKLATVALA Nature Vol. 322 - 7 août 1986 - p. 547 à 549
(2) "Mecanism of macrophage activation in RHUMATOID ATHRITIS, the role of IFNγ"M.G. RIDLEY et coll. dans Clinical and experimental immunology 1986 Vol. 63 p. 587 à 593
(3)
a B. KOCK, W. REGNAT, J. Clin. Lab. Immunol. (1984), 13, 171,178
b CARDENAS, MARSHALL, Journal of Immunological methods, 89 (1986) 181-189
(4) DANADERKA - The Lancet, November 23, 1875, p. 1190 CACHECTIN/TUMOUR NECROSIS FACTOR
(5) OPPENHEIM et al. properties of IL-1 Federation proceedngs Vol. 41, n°2, February 1982.
(6) VAITUKAITIS et al. - J. Clin. Endocrinol. Metab. 33, 988-911 : 1971
(7) GREENWOOD, Biochem. J. 8 : 114-120, 1963
(8) KOHLER of MILSTEIN Nature Vol. 256, 1975.

**Revendications**

1. Dosage immunologique d'une monokine d'un échantillon sanguin, notamment du TNFα ou de l'IL-1β caractérisé en ce qu'on dose la monokine dans le sérum ou le plasma obtenu à partir du sang total stimulé par un agent mitogène préalablement à toute séparation entre le plasma ou le sérum et les cellules sanguines, mais sans étape de culture cellulaire des cellules sanguines.

2. Dosage de monokine, selon la revendication 1., caractérisé en ce que le sang est prélevé sur un anti-coagulant, tel que l'EDTA, l'héparine ou le citrate, puis mis en contact avec l'agent mitogène et laissé à incuber, puis décanté notamment par centrifugation, le dosage de la monokine ayant lieu dans le plasma ainsi obtenu.

3. Dosage, selon l'une des revendications 1 ou 2., caractérisé en ce que l'agent mitogène est de la phytohémagglutinine (PHA) ou un lipopolysaccharide (LPS).

4. Dosage, selon l'une des revendications 1 à 3., caractérisé en ce que la stimulation directe du sang total se fait avec 0,02 à 50µg/ml de PHA ou 10 à 220µ/ml de LPS, une incubation comprise entre 30 minutes et 48 heures et à une température comprise entre 4°C et 40°C.

5. Dosage, selon l'une des revendications précédentes, caractérisé en ce que la stimulation du sang total se fait avec des concentrations de 1 à 10µg/ml de PHA ou 15 à 75µg/ml de LPS.

6. Dosage, selon l'une des revendications précédentes, caractérisé en ce que la stimulation du sang total se fait avec 5µg/ml de PHA ou 25µg/ml de LPS pour une incubation de 2 heures à 37°C.

7. Dosage immunologique du TNF ou de l'IL-1 selon l'une des revendications précédentes caractérisé en ce qu'on effectue un dosage par déplacement notamment du type RIA en utilisant un antisérum polyclonal de lapin anti-TNFα ou anti-IL-1β respectivement ne présentant pas de réactions croisées avec les autres cytokines.

8. Dosage immunologique de monokine, selon l'une des revendications précédentes, caractérisé en ce qu'on effectue un dosage immunométrique, notamment du type IRMA à l'aide d'anticorps monoclonaux dirigés contre ladite monokine ne présentant pas de réactions croisées avec les autres cytokines.

9. Trousse de dosage immunologique de monokines, selon l'une des revendications précédentes, caractérisé en ce que la trousse comporte un agent mitogène.

10. Trousse de dosage selon la revendication 12. caractérisée en ce que l'agent mitogène est contenu dans le tube de prélèvement sous vide de l'échantillon de sang à doser qui comporte également de l'anticoagulant.

**Patentansprüche**

1. Immunologische Bestimmung eines Monokins von einer Blutprobe, insbesondere von TNFα oder IL-1β, dadurch gekennzeichnet, daß man das Monokin in dem Serum oder dem Plasma bestimmt, das ausgehend von zuvor vollständig durch ein mitogenes Mittel stimuliertem Blut erhalten wurde, bei dem eine vollständige Trennung zwischen dem Serum oder dem Plasma und den Blutzellen erfolgte, jedoch ohne Stufe einer Zellkultur der Blutzellen.

2. Bestimmung von Monokin nach Anspruch 1, dadurch gekennzeichnet, daß das Blut über einem Antikoagulans wie EDTA, Heparin oder Citrat entnommen wird, danach mit einem mitogenen Mittel in Kontakt gebracht und inkubieren gelassen wird, anschließend insbesondere durch Zentrifugieren dekantiert wird und die Bestimmung des Monokins in dem auf diese Weise erhaltenen Plasma stattfindet.

3. Bestimmung nach einem der Ansprüche 1 oder 2, dadurch gekennzeichnet, daß das mitogene Mittel Phytohämagglutinin (PHA) oder ein Lipopolysaccharid (LPS) ist.

4. Bestimmung nach einem der Ansprüche 1 bis 3, dadurch gekennzeichnet, daß die direkte Stimulierung des gesamten Blutes mit 0,02 bis 50 µg/ml PHA oder 10 bis 220 µg/ml LPS erfolgt, woran sich eine Inkubation während einer Zeitdauer zwischen 30 Minuten und 48 Stunden bei einer Temperatur zwischen 4 °C und 40 °C anschließt.

5. Bestimmung nach einem der vorstehenden Ansprüche, dadurch gekennzeichnet, daß die Stimulierung des gesamten Blutes mit Konzentrationen von 1 bis 10 µg/ml PHA oder 15 bis 75 µg/ml LPS erfolgt.

6. Bestimmung nach einem der vorstehenden Ansprüche, dadurch gekennzeichnet, daß die Stimulierung des gesamten Blutes mit 5 µg/ml PHA oder 25 µg/ml LPS für eine Inkubation von 2 Stunden bei einer Temperatur von 37 °C erfolgt.

7. Immunologische Bestimmung von TNFα oder IL-1β nach einem der vorstehenden Ansprüche, dadurch gekennzeichnet, daß man eine Bestimmung durch Verschiebung insbesondere des Typs RIA durchführt, unter Verwendung eines polyklonalen Antiserums des Kaninchens, jeweils anti-TNFα oder anti-IL-1β, das keine Kreuzreaktionen mit den anderen Cytokinenen aufweist.

8. Immunologische Bestimmung von Monokin nach einem der vorstehenden Ansprüche, dadurch gekennzeichnet, daß man eine immunometrische Bestimmung insbesondere des Typs IRMA durchführt, mit Hilfe von gegen das genannte Monokin, das keine Kreuzreaktionen mit den anderen Cytokinenen aufweist, gerichteten monoklonalen Antikörpern.

9. Besteck zur immunologischen Bestimmung von Monokinen nach einem der vorstehenden Ansprüche, dadurch gekennzeichnet, daß das Besteck ein mitogenes Mittel umfaßt.

10. Bestimmungsbesteck nach Anspruch 9, dadurch gekennzeichnet, daß das mitogene Mittel in dem Rohr zur Entnahme der zu bestimmenden Blutprobe mittels Unterdruck enthalten ist, das auch das Antikoagulans umfaßt.

**Claims**

1. An assay of a monokine in a blood sample, in particular of TNFα or of IL-1β, wherein the monokine is assayed in the serum or plasma obtained from whole blood stimulated with a mitogenic agent prior to any separation between plasma or serum and the blood cells, without any blood cell culturing step.

2. The monokine assay as claimed in claim 1, wherein the blood is withdrawn onto an anticoagulant, such as EDTA, heparin or citrate, then brought into contact with the mitogenic agent and left to incubate, and then clarified, in particular, by centrifugation, the assay of the monokine taking place in the plasma thereby obtained.

3. The assay as claimed in one of claims 1 or 2, wherein the mitogenic agent is a phytohemagglutinin (PHA) or a lipopolysaccharide (LPS).

4. The assay as claimed in one of claims 1 to 3, wherein the direct stimulation of whole blood is carried out with 0.02 to 50 µg/ml of PHA or 10 to 200 µg/ml of LPS, an incubation of between 30 minutes and 48 hours and at a temperature of between 4°C and 40°C.

5. The assay as claimed in one of the preceding claims, wherein the stimulation of whole blood is carried out with concentrations of 1 to 10 µg/ml of PHA or 15 to 75 µg/ml of LPS.

6. The assay as claimed in one of the preceding claims, wherein the stimulation of whole blood is carried out with 5 µg/ml of PHA or 25 µg/ml of LPS for an incubation of 2 hours at 37°C.

7. An immunoassay of TNFα or of IL-1β as claimed in one of the preceding claims, wherein a displacement assay, in particular of the RIA type, is performed using, a rabbit polyclonal anti-TNFα or anti-IL-1β antiserum, respectively, not showing cross-reactions with other cytokines.

8. The immunoassay of monokine as claimed in one of the preceding claims, wherein an immunometric assay, in particular of the IRMA type, is performed using monoclonal antibodies directed towards the said monokine, not showing cross-reactions with other cytokines.

9. Monoclonal antibodies which are useful, in particular, for the immunoassays as claimed in one of the preceding claims, wherein the antibodies are secreted by hybridomas produced by the fusion of mouse spleen cells and SP2-0 myeloma cells.

10. A kit for the immunoassay of monokines a claimed in one of the preceding claims, wherein the kit contains, among other reagents, a mitogenic agent.

FIG_1

FIG. 2

FIG_3

FIG.4

$\dfrac{B}{B_o} \times 100$

FIG_5